# EUROPEAN PATENT SPECIFICATION

(11) **EP 3 723 859 B1**
(45) Date of publication and mention of the grant of the patent: **30.08.2023**
(21) Application number: 17851874.2
(22) Date of filing: 11.12.2017
(51) Int. Cl.: A61K 31/585, A61P 25/28, C07J 71/00, C07J 41/00

(54) **COMPOUNDS FOR TREATING NEURODEGENERATIVE DISORDERS**
VERBINDUNGEN ZUR BEHANDLUNG VON NEURODEGENERATIVEN ERKRANKUNGEN
COMPOSÉS POUR LE TRAITEMENT DE TROUBLES NEURODÉGÉNÉRATIFS

(43) Date of publication of application: 21.10.2020
(73) Proprietor: Ethnodyne, 75008 Paris (FR); CENTRE NATIONAL DE LA RECHERCHE SCIENTIFIQUE -CNRS-, 75016 Paris (FR)
(72) Inventor: RABHI, Chérif, 91220 BRETIGNY SUR ORGE (FR); CARIEL, Léon, 75006 PARIS (FR); DA COSTA NOBLE, Christian, 75008 PARIS (FR); OUAZZANI, Jamal, 91300 MASSY (FR); ARCILE, Guillaume, 91940 LES ULIS (FR); LE GOFF, Géraldine, 91940 Les Ulis (FR)
(74) Representative: Casalonga
(86) International application number: PCT/IB2017/001758
(87) International publication number: WO 2019/116070

(56) References cited:
- US-A1- 2016 091 504
- US-A1- 2017 022 247
- GOTTUMUKKALA V. SUBBARAJU ET AL: "Ashwagandhanolide, a Bioactive Dimeric Thiowithanolide Isolated from the Roots of Withania somnifera [bottom]", JOURNAL OF NATURAL PRODUCTS., vol. 69, no. 12, 1 December 2006 (2006-12-01), pages 1790-1792, XP055469994, US ISSN: 0163-3864, DOI: 10.1021/np060147p

## Description

The invention relates to compounds of formula (I), their method of synthesis as well as their use to treat neurodegenerative disorders.

Neurodegenerative disorders correspond to the disorders in the central nervous system that are characterized by the progressive loss of neural tissues. Neurodegenerative diseases are one of the most debilitating conditions and usually associated with mutated genes, accumulation of abnormal proteins, increased reactive oxygen species (ROS) or destruction of the neurons in a specific part of the brain. Changes in the neurons cause them to function abnormally and eventually result in the cells' demise. The reason is the inability of the neurons to regenerate on their own after the neural deterioration or severe damage. This leads to disorders like multiple sclerosis (MS), Amyotrophic lateral sclerosis (ALS) and Motor Neuron Diseases (MND). The incidence is expected to soar as the population ages, because neurodegenerative diseases strike primarily in mid-to late-life.

Multiple sclerosis (MS), also known as disseminated sclerosis or encephalomyelitis disseminata, is an inflammatory disease in which the insulating covers of nerve cells in the brain and spinal cord are damaged. This damage disrupts the ability of parts of the nervous system to communicate, resulting in a wide range of signs and symptoms, including physical, mental, and sometimes psychiatric problems. MS takes several forms, with new symptoms either occurring in isolated attacks (relapsing forms) or building up over time (progressive forms). Between attacks, symptoms may disappear completely. However, permanent neurological problems often occur, especially as the disease advances.

Multiple sclerosis is the most common autoimmune disorder affecting the central nervous system.

The cause of MS is not clear, but its pathology consists of immune infiltration into the central nervous system (CNS), inflammation, demyelination and finally axonal degeneration. MS is usually diagnosed based on the presenting signs and symptoms and the results of supporting medical tests.

Myelination in the CNS involves sequential developmental processes in which precursors of oligodendrocytes (OPCs) migrate, proliferate, and differentiate into newly formed oligodendrocytes (OL), after which those oligodendrocytes selected by target-dependent survival mechanisms wrap myelin membrane around the axons to form the sheath. Each oligodendrocyte can myelinate many axons, with the number of wraps proportional to the axon diameter and regulated tightly by reciprocal signaling between oligodendrocyte and axons.

So far, there is no known cure for multiple sclerosis. Treatments attempt to improve function after an attack and prevent new attacks.

Motor neuron diseases or disorders (MNDs) are characterized by progressive loss of motor neurons of the spinal cord ('lower motor neurons' (MN)) or motor neurons of the brain ('upper motor neurons'), or both, leading to atrophy and/or spasticity of the associated musculature. Spinal muscular atrophy (SMA), amyotrophic lateral sclerosis (ALS) and hereditary spastic paraplegia (HSP) are the most common MNDs.

When there are disruptions in the signals between the upper motor neurons and the lower motor neurons, the limb muscles develop stiffness (*spasticity*), movements become slow and effortful, and tendon reflexes such as knee and ankle jerks become overactive. Over time, the ability to control voluntary movements (such as speaking, walking, breathing, and swallowing) can be lost.

MNDs occur in adults and children. In children, particularly in inherited or familial forms of the disease, symptoms can be present at birth or appear before the child learns to walk. In adults, MNDs occur more commonly in men than in women, with symptoms appearing after age 40.

MNDs are classified according to whether they are inherited or sporadic, and to whether degeneration affects upper motor neurons, lower motor neurons, or both. The causes of most MNDs are not known.

Amyotrophic lateral sclerosis (ALS), also called Lou Gehrig's disease or classical motor neuron disease, is the most common form of MND, with both upper and lower motor neuron involvement. ALS affects both upper and lower motor neurons. It has inherited and sporadic forms and can affect the arms, legs, or facial muscles. Although the majority of ALS cases are sporadic, up to 10% are inherited (Robberecht & Philips, 2013) and the most common familial forms of ALS in adults are caused by mutations of the superoxide dismutase gene, or SOD1, located on chromosome 21. There are also rare juvenile-onset forms of familial ALS. This form of the disease is characterized by weakness and wasting in the limbs. Muscle weakness and atrophy occur on both sides of the body. Affected individuals lose strength and the ability to move their arms and legs, and to hold the body upright. Other symptoms include spasticity, spasms, muscle cramps, and fasciculations. Speech can become slurred or nasal. When muscles of the diaphragm and chest wall fail to function properly, individuals lose the ability to breathe without mechanical support. Although the disease does not usually impair a person's mind or personality, several recent studies suggest that some people with ALS may develop cognitive problems involving word fluency, decision-making, and memory. Most individuals with ALS die from respiratory failure, usually within 3 to 5 years from the onset of symptoms.

Progressive bulbar palsy (PBP) involves both the upper and lower motor neurons. Symptoms include pharyngeal muscle weakness (involved with swallowing), weak jaw and facial muscles, progressive loss of speech, and tongue muscle atrophy. Limb weakness with both lower and upper motor neuron signs is almost always evident but less prominent. Affected persons have outbursts of laughing or crying (called *emotional lability*)*.* In about 25 % of individuals with ALS, early symptoms begin with bulbar involvement. Some 75 % of individuals with classic ALS eventually show some bulbar involvement. Life expectancy is between six months and three years from onset of symptoms.

Progressive muscular atrophy (PMA) affects only a small proportion of people, mainly causing damage to the lower motor neurons. Early symptoms may be noticed as weakness or clumsiness of the hand. Most people live for more than five years.

Primary lateral sclerosis (PLS) is a rare form of MND involving the upper motor neurons only, causing mainly weakness in the lower limbs, although some people may experience clumsiness in the hands or speech problems. It occurs when specific nerve cells in the motor regions of the cerebral cortex gradually degenerate, causing the movements to be slow and effortful. Difficulty with balance may lead to falls. PLS is more common in men than in women, with a very gradual onset that generally occurs between ages 40 and 60. The cause is unknown. The symptoms progress gradually over years, leading to progressive stiffness and clumsiness of the affected muscles. The disorder is not fatal but may affect quality of life, if it develops into ALS.

Spinal muscular atrophy (SMA) is an autosomal, hereditary recessive disorder caused by defects in the gene SMN1. In SMA, insufficient levels of the SMN protein lead to degeneration of the lower motor neurons, producing weakness and wasting of the skeletal muscles. This weakness is often more severe in the trunk and upper leg and arm muscles than in muscles of the hands and feet.

Kennedy's Disease, also known as progressive spinobulbar muscular atrophy, is an X-linked recessive progressive disorder of the motor neurons caused by mutations in the gene for the androgen receptor. Symptoms include weakness and atrophy of the facial, jaw, and tongue muscles, leading to problems with chewing, swallowing, and changes in speech. Early symptoms may include muscle pain and fatigue. Individuals with Kennedy's disease also develop sensory loss in the feet and hands. It only affects men, but women may carry the mutation. The course of the disorder is generally slowly progressive. Individuals tend to remain ambulatory until late in the disease. The life expectancy for individuals with Kennedy disease is usually normal.

Post-polio syndrome (PPS) is a condition that can strike polio survivors decades after their recovery from poliomyelitis. Polio is an acute viral disease that destroys motor neurons. PPS and Post-Polio Muscular Atrophy (PPMA) are thought to occur when the surviving motor neurons are lost in the aging process or through injury or illness. Symptoms include fatigue, slowly progressive muscle weakness, muscle atrophy, fasciculations, cold intolerance, and muscle and joint pain. These symptoms appear most often among muscle groups affected by the initial disease, and may consist of difficulty breathing, swallowing, or sleeping. PPS is not usually life threatening. Doctors estimate that 25 to 50 percent of survivors of paralytic poliomyelitis usually develop PPS.

Multifocal motor neuropathy (MMN) is a progressively worsening condition where muscles in the extremities gradually weaken. MMN is thought to be autoimmune and involves only lower motor nerves. MMN usually involves very little pain however muscle cramps, spasms and twitches can cause pain for some sufferers. MMN is not fatal, and does not diminish life expectation.

Monomelic amyotrophy (MMA) is an untreatable, focal motor neuron disease that primarily affects young males in India and Japan. MMA is marked by insidious onset of muscular atrophy, which stabilizes at a plateau after two to five years from which it neither improves nor worsens.

Paraneoplastic motor neuron disease is a disease affecting the motor neurons.

Lambert-Eaton Myasthenic Syndrome (LEMS) is a rare autoimmune disorder characterized by muscle weakness of the limbs. Around 60% of those with LEMS have an underlying malignancy, most commonly small cell lung cancer; it is therefore regarded as a paraneoplastic syndrome.

Myasthenia gravis (MG) leads to fluctuating muscle weakness and fatigue. In the most common cases, muscle weakness is caused by circulating antibodies that block acetylcholine receptors at the postsynaptic neuromuscular junction, inhibiting the excitatory effects of the neurotransmitter acetylcholine on nicotinic receptors at neuromuscular junctions. Alternatively, in a much rarer form, muscle weakness is caused by a genetic inherited defect in some portion of the neuromuscular junction.

Botulism, a rare and potentially fatal illness caused by a toxin produced by the bacterium *Clostridium botulinum*, prevents muscle contraction by blocking the release of acetyl choline, thereby halting postsynaptic activity of the neuromuscular junction.

Hereditary spastic paraplegia (HSP) is the collective term for a group of clinically and genetically heterogeneous neurodegenerative disorders characterized by progressive spasticity and weakness in the lower limbs due to loss of upper motor neurons (Harding, 1983).

There is no cure or standard treatment for the MNDs. Symptomatic and supportive treatment can help people be more comfortable while maintaining their quality of life. Research has provided evidence about the role of excitotoxicity in the pathophysiology of sporadic amyotrophic lateral sclerosis and suggests that glutamate receptors activation contributes greatly in mediating injury to motor neurons.

Thus, there is a clear need for alternative therapies that prevent progression and hopefully reverse the effects of neurodegenerative diseases on human.

US 2017/022247 A1, GOTTUMUKKALA V. SUBBARAJU ET AL., JOURNAL OF NATURAL PRODUCTS., vol. 69, no. 12, 1 December 2006 (2006-12-01), pages 1790-1792 and US 2016/091504 A1, respectively, refer to structurally different analogs of withanolide and their use in treatment of neurodegenerative diseases.

Surprisingly, the applicant has found that, by using a specific compound, it is possible to treat neurodegenerative disorders.

The purpose of the invention is therefore to use a compound of formula (I) 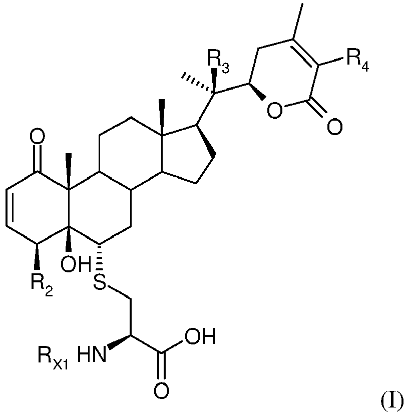 in which
R2 is H, OH, (CH₂)n-CH₃, with n=2, 4 or 6, a glucopyranose or a glucofuranose;
R3 is H, OH, CH₂OH or a glucofuranose;
R4 is H, OH, CH₃, CH₂OH, a glucofuranose, C₆H₅, C₁₀H₇, C₆H₄X or C₁₀H₆X with X= F, Cl, Br or I;
Rxi is H or an aminoacid chosen among tryptophane, lysine, methionine, phenylalanine, threonine, valine, leucine, isoleucine, arginine or histidine
for the treatment or prevention of neurodegenerative disorders.

The invention is also relative to the synthesis of this compound.

Other objects, features, aspects and advantages of the invention will appear more clearly on reading the description and examples that follow:

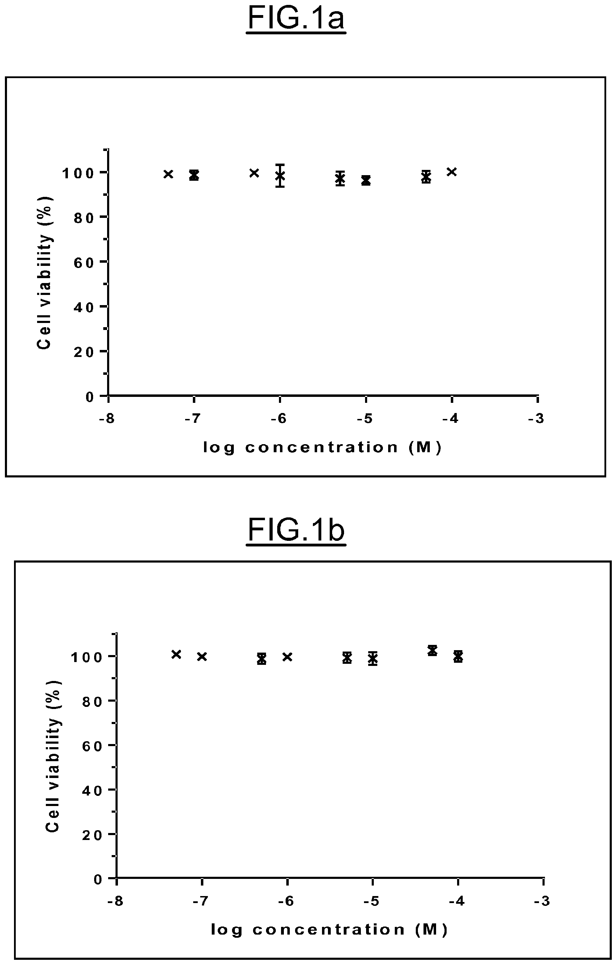

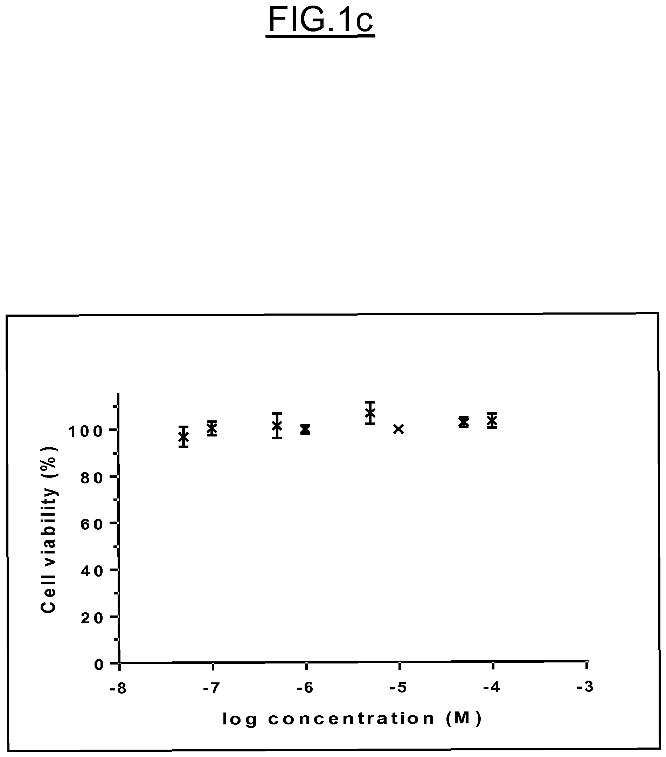

Figure 1: cytotoxicity tests with compound CR591 on several cell lines. a=MRC5, b=HCT116, c=HUVEC.

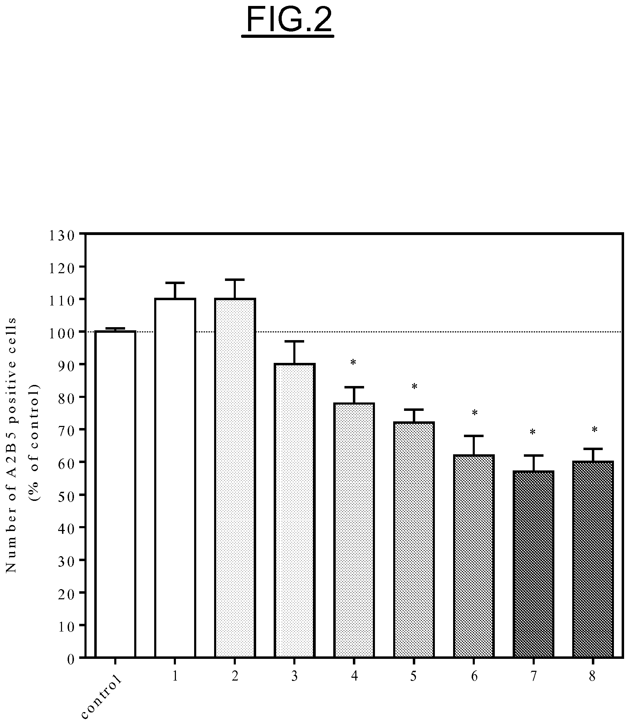

Figure 2: Effect of CR591 on OPC number after 12 days of treatment. Bar 1: CR591 1 pmol/L, bar 2: CR591 10 pmol/L, bar 3: CR591 100 pmol/L, bar 4: CR591 1 nmol/L, bar 5: CR591 10 nmol/L, bar 6: CR591 100 nmol/L, bar 7: CR591 1 µmol/L, bar 8: CR591 10 µmol/L. Data were expressed as percentage of control as mean +/-SEM (100% = no compound). Statistical analyses were performed using the Graph pad prism for one-way ANOVA followed by PLSD fisher's test. * p< 0.05 was considered significant.

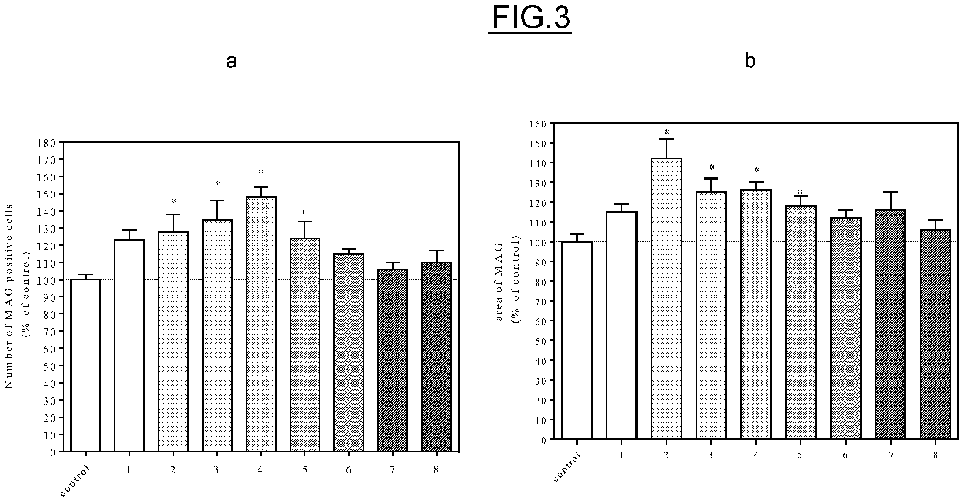

Figure 3: Effect of CR591 on a) the number of MAG positive cells (OL) and b) the area of MAG expression after 18 days of treatment. Bar 1: CR591 1 pmol/L, bar 2: CR591 10 pmol/L, bar 3: CR591 100 pmol/L, bar 4: CR591 1 nmol/L, bar 5: CR591 10 nmol/L, bar 6: CR591 100 nmol/L, bar 7: CR591 1 µmol/L, bar 8: CR591 10 µmol/L. Data were expressed as percentage of control as mean ± SEM (100% = no compound). Statistical analyses were performed using the Graph pad prism for one-way ANOVA followed by PLSD fisher's test. ^{∗} p< 0.05 was considered significant.

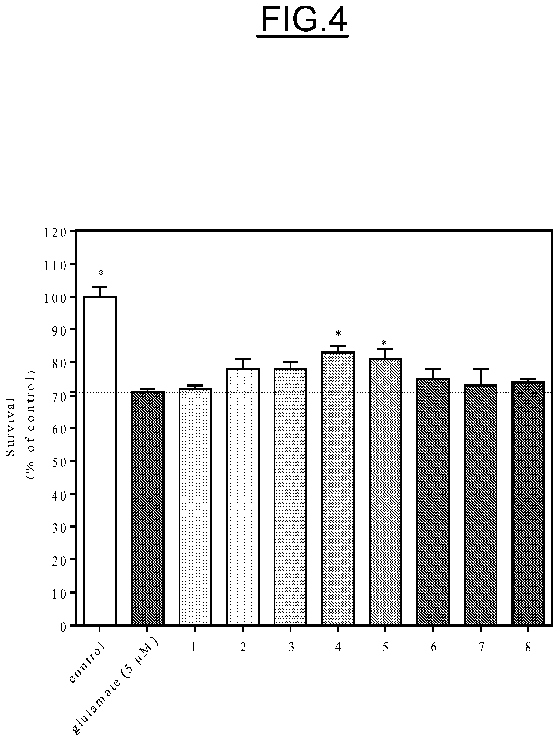

Figure 4: Effect of CR591 on neuron survival of primary rat motor neurons injured with glutamate (48h, 5 µM). Bar 1: CR591 1 pmol/L, bar 2: CR591 10 pmol/L, bar 3: CR591 100 pmol/L, bar 4: CR591 1 nmol/L, bar 5: CR591 10 nmol/L, bar 6: CR591 100 nmol/L, bar 7: CR591 1 µmol/L, bar 8: CR591 10 µmol/L. Data were expressed as percentage of control as mean ± SEM (100% = no glutamate, no compound). Statistical analyses were performed using the Graph pad prism for one-way ANOVA followed by PLSD fisher's test. * p< 0.05 was considered significant.

The invention is directed to a compound of formula (I) 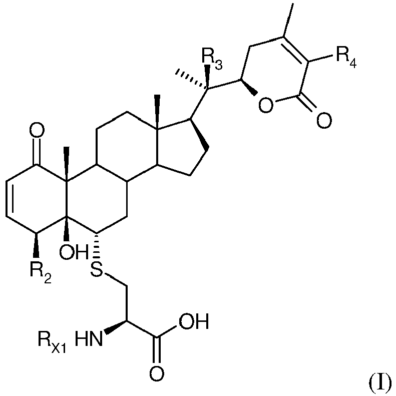 in which
R2 is H, OH, (CH₂)n-CH₃, with n=2, 4 or 6, a glucopyranose or a glucofuranose;
R3 is H, OH, CH₂OH or a glucofuranose;
R4 is H, OH, CH₃, CH₂OH, a glucofuranose, C₆H₅, C₁₀H₇, C₆H₄X or C₁₀H₆X with X= F, Cl, Br or I;
Rxi is H or an aminoacid chosen among tryptophane, lysine, methionine, phenylalanine, threonine, valine, leucine, isoleucine, arginine or histidine
as well as its use for the treatment of neurodegenerative disorders in a mammal. Preferably, the mammal is a human.

Preferably in the compound of formula (I), the glucopyranose and the glucofructose are D glucopyranose and D glucofructose.

Preferably, in the compound of formula (I), R2 is H, or OH ; R3 is H, OH or CH₂OH; R4 is H, OH, CH₃, CH₂OH and Rxi is H or an aminoacid chosen among tryptophane, lysine, methionine, phenylalanine, threonine, valine, leucine, isoleucine, arginine or histidine.

More preferably, in the compound of formula (I), R2 is OH, R3 is H, R4 is CH₂OH and R_{X1} is H, giving the compound CR591 of formula:

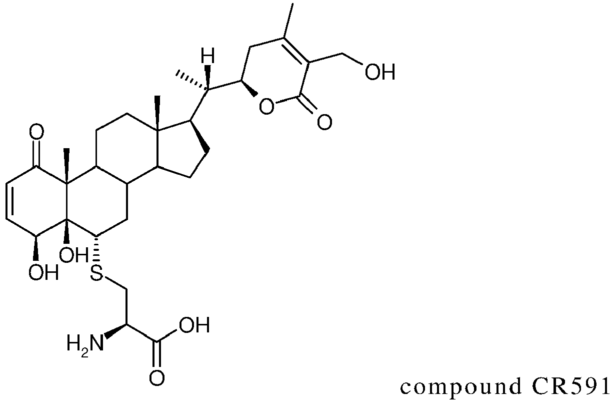

The method of synthesis of the compound of formula (I) comprises the reaction between a compound of formula (II) in which the substituent R1 is H, and R2, R3, R4 have the same meaning as in formula (I), 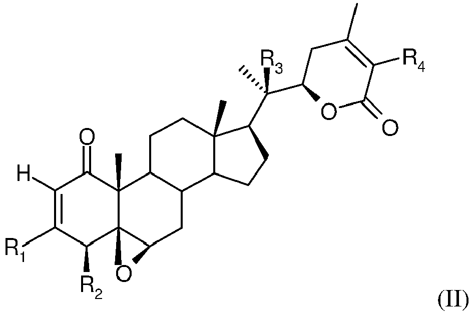 and a compound of formula (III) in which the substituent R_{X1} has the same meaning as in formula (I) 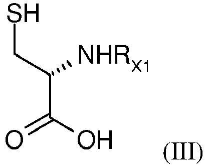 to obtain the compound of formula (I).

Preferably, the reaction is performed in the presence of at least a solvent. Preferably, the solvent used is tetrahydrofurane and/or water. More preferably, the solvent is a mixture of tetrahydrofurane and water.

Preferably, the reaction is performed at a temperature comprised between 40 and 80°C. More preferably, the temperature is 65°C. Preferably, the reaction last from 8h to 15h.

The compounds of formula (I) according to the invention are useful as medicaments.

The compounds of formula (I) can be used to treat, prevent or limit neurodegenerative disorders in a mammal, preferably a human.

The compounds of formula (I) according to the invention can be used to treat or limit development of demyelinating diseases in a mammal, preferably a human.

Preferably, the demyelinating diseases comprise multiple sclerosis, acute disseminated encephalomyelitis, adrenoleukodystrophy, adrenomyeloneuropathy, Leber's Hereditary Optic Atrophy and related mitochondrial disorders, HTLV-associated Myelopathy and diseases linked to demyelination of PNS nerves.

In certain embodiments of the invention, the compounds of the invention reduce the progression of MS in particular.

As used herein, "treating" MS means providing any clinical benefit to a subject with MS. The clinical benefit may be temporary or long-lasting. In various non-limiting embodiments, the treatment results in one or more clinical outcome selected from the group consisting of:
(a) decrease in MS disease progression;
(b) decrease in MS disease severity;
(c) decrease in nerve cell demyelination:
(d) decrease in frequency or severity of relapsing MS attacks;
(e) decrease in MS clinical symptoms;
(f) healing of damaged nerve tissue (neuro-restoration);
(g) increase in remyelination of demyelinated nerves in the central nervous system (neuro-restoration);
(h) protection of damaged nerve tissue from further disease activity (neuroprotection) :
(i) promoting neuronal outgrowth (neuro-regeneration) in the central nervous system; and
(j) decrease in disability caused by MS.

As used herein, "limiting development" of MS means providing a limitation in development of symptoms or disease in a subject that is at risk of developing MS. Exemplary subjects at risk of MS include, but are not limited to subjects with a relative (identical twin, non-identical twin, sibling, parent, etc.) that has MS and subjects that, have suffered a clinically isolated syndrome (CIS), which is a subject's first neurological episode, caused by inflammation or demyelinisation of nerve tissue.

The compound of formula (I) according to the invention can be used to treat or limit development of neuromuscular diseases in a mammal, preferably a human.

Preferably, the neuromuscular diseases comprise MN diseases, ALS, PBP, PMA, PLS, SMA, Kennedy's disease, PPS, PPMA, MMN, MMA, paraneoplastic motor neuron disease, LEMS, MG and botulism.

The compounds of formula (I) according to the invention are therefore used to treat or limit development of MN diseases like ALS (amyotrophic lateral sclerosis), PBP (progressive bulbar palsy), PMA (progressive muscular atrophy), PLS(primary lateral sclerosis), SMA (spinal muscular atrophy), Kennedy's disease, PPS (Post-polio syndrome), PPMA (Post-Polio Muscular Atrophy), MMN (Multifocal motor neuropathy), MMA (Monomelic amyotrophy), paraneoplastic motor neuron disease, LEMS (Lambert-Eaton Myasthenic Syndrome), MG (Myasthenia gravis) and botulism, in particular by limiting degeneration of motor neurons.

In certain embodiments of the invention, compound of formula (I) treat, limit development or reduce the progression of ALS in particular.

As used herein, "treating" ALS means providing any clinical benefit to a subject with ALS. The clinical benefit may be temporary or long-lasting. In various non-limiting embodiments, the treatment results in one or more clinical outcome selected from the group consisting of:
(a) decrease in ALS disease progression;
(b) decrease in ALS disease severity;
(c) decrease in ALS clinical symptoms;

As used herein, "reducing the progression" or "limiting development" of ALS means providing a limitation in development of symptoms or disease in a subject that is at risk of developing ALS.

The neuromuscular diseases comprise MN diseases, ALS, PBP, PMA, PLS, SMA, Kennedy's disease, PPS, PPMA, MMN, MMA, paraneoplastic motor neuron disease, LEMS, MG and botulism.

The compound of formula (I) according to the invention is formulated for oral or parenteral administration.

A person skilled in the art of pharmaceutical formulation will implement the various useful forms for administration of the formulations of the invention. The formulations containing the compound of formula (I) of the invention may be in liquid, gel, emulsion, solid or injectable form.

These formulations used may additionally include suspensions, emulsions, syrups containing conventionally used inert diluents, and possibly other substances such as wetting agents, sweeteners, preservatives, thickeners, colourings or any other substance known to a person skilled in the art suitable for oral administration, in particular ((sodium sorbate (E201) (Sigma-Aldrich), anthocyanin (E163) (FBC Industries, USA), sodium metabisulphite (E223) (Sigma-Aldrich), alpha-tocopherol (E307) (FBC Industries, USA).

The formulations used may also comprise solvents or other excipients such as water, propylene glycol, vegetable oils or other suitable organic solvents.

The term "excipient" is used to mean any compound which does not interfere with the effectiveness of the biological activity of the formulation according to the invention, and which is not toxic to the host to which it is administered.

The formulation used may also contain adjuvants, such as wetting agents, isotoning agents, emulsifiers, salts or any other substances known to a person skilled in the art that can be used as adjuvants (Polydimethylsiloxane, polyvinyl alcohol (PVA), hydrogels (Carbopol), polyvinylpyrrolidone, hydroxypropyl cellulose (HPC), poloxamer 188, EDTA, chlorobutanol) (Lubrizol, France, Dow Corning, USA).

Advantageously, the formulation may comprise other substances such as vitamins, mineral salts, a pharmaceutically acceptable vector, stabilisers, antioxidants, or any other substance known to a person skilled in the art and intended to be integrated into a drug.

Preferably, the formulation is liquid, orally administrable and contains at least a compound of formula (I), some preservatives, vitamins, water and salt. More preferably, the preservatives are potassium sorbate or benzoate. The vitamin is riboflavin (vitamin B2).

The therapeutic formulation containing the compound of formula (I) of the invention and used in the method of the invention is administered in a pharmaceutically acceptable vehicle.

The terms "pharmaceutically acceptable vehicle" is used to mean any vehicle which does not interfere with the effectiveness of the biological activity of the formulation according to the invention and which is not toxic to the host to which it is administered.

The formulation obtained is usable as a medicinal product for a mammal, and more particularly for humans, to assist in the treatment, prevention or limitation of development of neurodegenerative disorders.

The term "medicinal product" is used to mean a product containing an accurate dose of said preparation according to European directive 65/65/EC, namely any substance or composition described as possessing curative or preventive properties with respect of human or animal disease. For example, the medicinal product containing said preparation at therapeutic doses can be administered orally as a capsule or a tablet, or injected via any other route to confer the beneficial effects.

An appropriate dosage of the therapeutic formulation can be determined by one of skill in the art, taking into consideration the findings described herein together with typical factors such as the body mass of the patient, the physical condition of the patient, and so on. The dosage should contain the therapeutic formulation in an amount that is effective for treating, preventing or limiting development of neurodegenerative disorders, including demyelinating diseases and in particular MS and MNDs and in particular ALS.

The drug can be administered daily, weekly, or on an intermittent basis. For example, the drug can be administered for three weeks on, followed by one week off, or for two weeks on, followed by one week off, or under other dosing schedules as can be determined by one skilled in the field.

The particular dose selected will depend upon the mode of administration and dosing regimen selected. One preferred schedule is a once daily oral dosing schedule. When longer periods of time are prescribed between each application (typically the case for i.v. administration), each unit dose may be larger than when daily dosages are provided.

The daily dose of the compounds of the invention used may vary according to the needs and severity of symptoms of the patient and according to the route. Typically, the daily dose is between 10 mg/mL and 300 mg/mL of the compound.

Preferably, the daily dose for an adult human is between 30 and 100 mg/mL of the compound of formula (I).

The present invention will be explained in further detail by way of non-limiting examples below, which make reference to the appended drawings. The following methods were used in the experiments described in the examples that follow the description of the methods.

### Example 1: Synthesis of the compound CR591

In a 100 mL two-neck round-bottom flask, 625 mg (3.5 × 10⁻³ moles) of L-Cysteine hydrochloride monohydrate are solubilized in 20 ml of water, then a solution of withaferin A (Sigma Aldrich France) (70 mg , 1.4 × 10⁻⁴ mol) in THF (30 mL) is slowly added. Under inert atmosphere, the reaction mixture is brought to 65° C overnight.

The reaction mixture is concentrated under vacuum until THF is removed, the residual aqueous phase is added with 15 g of Amberlite XAD 16 resin and this heterogeneous mixture is left under strong stirring for 1 hour.

The mixture is separated by filtration, the resin is rinsed with 150 ml of water and then desorbed with then desorbed with methanol (2 × 50 mL).

The aqueous phase contains excess cysteine.

The organic phase is concentrated under reduced pressure to yield a colorless oil which will be purified by High Pressure Liquid Chromatography (HPLC).

14.7 mg of a white solid are obtained, ie a yield of 56.5% (compound CR591).

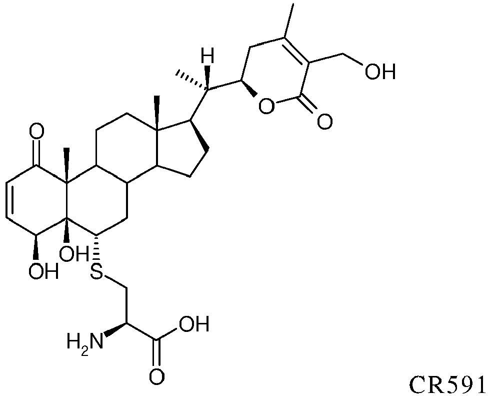

The sample was analyzed on an analytical HPLC device equipped with a 3.5 µm Sunfire III C18 (4.6 × 150 mm) reverse phase column (Waters), an Alliance^{®} Waters W2695 HPLC chain equipped with a Waters 2996 PDA detector. This chromatographic system is coupled to a Waters 2424 evaporative light scattering detector (DEDL). The HPLC system is controlled by Empower 3 software (Waters).

The solvents used are composed of ultrapure water (Merck Millipore Q-Gard 1 purification cartridge) + 0.1% Formic acid (VWR), acetonitrile (Carlo Erba SDS, HPLC grade, France) + 0.1% Formic acid. The standard gradient used is from 0 to 100% acetonitrile in 40 min + 10 min to 100% acetonitrile (total duration 50 min). The flow rate is 0.7 mL / min and the injection volume is 20 to 100 µL depending on the sample.

For mass spectrometry, HPLC-MS analyzes are performed on an Alliance^{®} Waters HPLC chain coupled to a Waters 2998 PDA-type UV detector, a DEDL Waters 2420 light scattering detector and a Micromass^{®} ZQ mass detector ( Waters).

The solvents are ultrapure HPLC water (Merck Millipore Q-Gard 1 purification cartridge) + 0.1% Formic Acid and acetonitrile (Carlo Erba SDS, HPLC grade) + 0.1% Formic Acid. The standard gradient used is from 0 to 100% acetonitrile in 40 min + 10 min to 100% acetonitrile (total duration 50 min). The flow rate is 0.7 mL / min and the injection volume is 20 to 100 µL depending on the sample. The samples used for HPLC analysis are filtered through 0.45 microns (Ait-France, ref: SFNY 013045N). The compounds are isolated by semi-preparative HPLC on an Alliance^{®} Waters HPLC chain (previously mentioned parameters) equipped with a Sunfire III C18 (10 × 250 mm) 5 µm reverse phase column (Waters). The standard gradient used is 20 to 45% acetonitrile in 40 min. The flow rate is 4 mL / min and the injection volume is about 200 µL.

The nuclear magnetic resonance experiments were carried out on 300, 500 and 600 MHz Bruker Avance devices using as the solvent deuterated methanol CD3OD (EurisoTop, France). Chemical shifts are expressed in ppm (parts per million) and calibrated against the reference solvent. The coupling constants are expressed in Hertz (Hz). The multiplicity of signals is expressed by the following abbreviations: s (singlet), ls (wide singlet), d (doublet), dd (doublet of doublets), t (triplet), m (multiplet), q (quadruplet). The signaling of protons and carbons was carried out from ¹D ¹H and ¹³C one-dimensional experiments, and two-dimensional 2D ¹H-¹H COSY, ¹H-¹³C HSQC or HMQC, ¹H-¹³C HMBC, ¹H-¹H ROESY. NMR spectra are processed using TopSpin (Brucker) dedicated software.

The high resolution mass spectra were performed on a mass spectrometer equipped with an electrospray and a TOF time-of-flight type mass analyzer (LCT^{®}, Waters).

Optical rotations of the compound was measured using a JascoTM P1010 polarimeter equipped with Spectro Manager software. The monochromatic light source is the sodium D line. The experiments were carried out with a 100 mm quartz tank of 350 µL, and the products were solubilized in methanol.

The infrared (IR) adsorption spectra of the described compound was measured on the Perkin-Elmer Spectrum 100 FT-IR spectrometer. The device is equipped with Spectrum software (version 6.3.5) from Perkin-Elmer. The compounds were prepared in solution in methanol and then dried with compressed air. The absorption bands are given in cm-1.

Elemental analysis is carried out on a Vario ELIII apparatus, with a detection of catharometry type for Carbon, Hydrogen, Nitrogen and Oxygen elements. For the determination of Sulfur, detection is carried out using infra-red.
NMR ¹H and ¹³C ( 500 MHz, Methanol -*d*₄)

| Position | *δ*_{C} | *δ*_{H} (mult; *J* in Hz) | HMBC |
|---|---|---|---|
| 1 | 204.1 | | |
| 2 | 127.5 | 5.89 (1H; dd; 10.2; 2.0) | C-4, C-10 |
| 3 | 148.8 | 6.58 (1H; dd; 10.2, 1.9) | C-1, C-5 |
| 4 | 67.3 | 4.89 (1H; m) | C-1, C-2, C-3, C-6 |
| 5 | 81.4 | - | |
| 6 | 52.4 | 3.02 (1H; d; 12.3) | C-4, C-5, C-7, C-10, Cvs (35,3) |
| 7 | 38.7 | 2.19 (1H; m), 1.55 (1H; m) | C-6, C-8, C-9, C-14 |
| 8 | 36.7 | 1.67 (1H; m) | |
| 9 | 47.0 | 1.39 (1H; m) | |
| 10 | 59.3 | - | |
| 11 | 28.4 | 1.81 (1H; m), 1.42 (1H; m) | |
| 12 | 40.5 | 1.14 (1H; m) | |
| 13 | 44.4 | - | |
| 14 | 56.3 | 1.18 (1H; m) | C-15 |
| 15 | 25.2 | 1.70 (1H; m), 1.27 (1H; m) | C-14 |
| 16 | 24.3 | 1.39 (1H; m), 0.90 (1H; m) | C-13 |
| 17 | 53.1 | 1.23 (1H; m) | |
| 18 | 12.4 | 0.75 (3H; s) | C-12, C-13, C14, C-17 |
| 19 | 10.5 | 1.25 (3H; s) | C-1, C-5, C-9, C-10 |
| 20 | 40.3 | 1.94 (1H; m) | |
| 21 | 13.6 | 0.99 (1H; d; 6.8) | C-17, C-20, C-22 |
| 22 | 80.1 | 4.44 (1H; d; 13.4) | C-20, C-24 |
| 23 | 30.8 | 2.52 (1H; d; 17.3), 2.16 (1H; m) | C-22, C-24, C-25 |
| 24 | 157.8 | - | |
| 25 | 126.4 | - | |
| 26 | 168.5 | - | |
| 27 | 56.5 | 4.34 (2H; m) | C-24, C-25, C-26 |
| 28 | 20.3 | 2.09 (3H; s) | C-23, C-24, C-25 |
| | | | |
| L-Cys | 35.3 | 3.13 (1H; dd; 14.3, 4.7), 2.95 (1H; dd; 14.3, 6.6) | C-6, Cys (55,7) |
| | 55.7 | 3.70 (1H; m) | Cys (35,3), Cys (172,8) |
| | 172.8 | - | |

High-resolution positif ion (HRMS): Calcd for C₃₁H₄₄NO₈S: 592,2911.
Found: 592,2910 [M+H]⁺
High-resolution negatif ion (HRMS): Calcd for C₃₁H₄₂NO₈S: 590,2846.
Found: 590,2742 [M-H]⁻
IR : 3363, 2936, 1670 cm⁻¹,

### Example 2 : Cytotoxicity tests on the product of example 1 according to the invention.

Human MRC-5 cell line derived from normal lung tissue and human HCT-116 colorectal carcinoma were obtained from the American Type Culture Collection (ATCC, Rockville, MD). Primary Human Umbilical Vein Endothelial Cells (HUVEC) isolated from the vein of the umbilical cord were obtained from Promocell (Germany). Cell lines were cultured according to the supplier's instructions. Human HCT-116 cells were grown in Gibco McCoy's 5A supplemented with 10% fetal calf serum (FCS) and 1% glutamine. Human MRC-5 cells were grown in DMEM supplemented with 10% fetal calf serum (FCS) and 1% glutamine. HUVECs were grown in Endothelial Cell Growth Medium 2 which is low-serum (2% V/V) media optimized for the cultivation of endothelial cells from large blood vessels.

Cells were counted using a Vi-cell XR (Beckman Coulter) and their viability assessed by 0.25% trypan blue dye exclusion. They were tested for the presence of mycoplasma before experiments with the Mycoplasma PCR Detection Kit (Applied Biological Materials Inc., Canada) in accordance with the manufacturer instructions and only mycoplasma-free cells were used for further investigations.

Cell lines were maintained at 37°C in a humidified atmosphere containing 5% CO₂. Cell growth inhibition was determined by an MTS assay according to the manufacturer's instructions (Promega, Madison, WI, USA). For IC₅₀ determination, the cells were seeded in 96-well plates (3 × 10³ cells/well) containing 100 *µ*L of growth medium. After 24 h of culture, the cells were treated with the compound CR591 of example 1 at 8 different final concentrations. Each concentration was obtained from serial dilutions in culture medium starting from the stock solution. Control cells were treated with the vehicle. Experiments were performed in triplicate.

After 72 h of incubation, 20 *µ*L of CellTiter 96^{®} AQᵤₑₒᵤₛ One Solution Reagent was added for 2h before recording absorbance at 490nm with a spectrophotometric plate reader. The dose-response curves were plotted with Graph Prism software and the IC₅₀ values were calculated using the Graph Prism software from polynomial curves (four or five-parameter logistic equations).

The results show that, whatever the cell line tested, the compound n°CR591 of example 1 does not show any toxicity at the concentrations tested (see Figures 1 a to c).

### Example 3: in vitro MS model

A reproducible *in vitro* myelination model based on primary cocultures of central neurons and oligodendrocytes culturing in 96-well plate is used and adapted to high throughput screening.

### a) Culture of neurons/oligodendrocytes

Neurons/oligodendrocytes were cultured as previously described by Charles et al., 2000. PNAS 97 7585-7590.

Briefly, pregnant Wistar female rats of 17 days gestation were killed by cervical dislocation (Janvier Labs, France) and the foetuses removed from the uterus. The forebrains were removed and placed in ice-cold medium of Leibovitz (L15; Pan Biotech, Germany) containing 2% of Penicillin-Streptomycin (Batch 1451013, PanBiotech) and 1% of bovine serum albumin (BSA) (Batch K180713, Pan Biotech). Cortexes were dissociated by trypsinisation for 20 min at 37°C, with Trypsin EDTA 1X (Batch 7310713, PanBiotech). The reaction was stopped by the addition of Dulbecco's modified Eagle's medium (DMEM) (Batch 9710913, PanBiotech) containing DNAase I grade II at 0.1 mg/mL (Batch H131108, PanBiotech) and 10% of foetal calf serum (Batch 41Q7218K, Invitrogen, France). Cells were then mechanically dissociated by 3 passages through a 10 mL pipette. Cells were centrifuged at 180 × g for 10 min at 4°C temperature on a layer of BSA (3.5%) in L15 medium. The supernatant was discarded and cells of the pellet were re-suspended in DMEM containing 10% of FCS. Cells were then centrifuged at 515 × g for 10 min at 4°C. The supernatant was discarded and cells of pellet were re-suspended in a culture medium consisting of Neurobasal (Batch 1625353, Invitrogen) supplemented with 2% of B27 (Batch 1618508, Invitrogen), 2 mM of L-glutamine (Batch 6620314, PanBiotech), 2% of PS solution, 1 % of foetal calf serum (FCS) and 10 ng/mL of platelet-derived growth factor (PDGF-AA) (Batch H131205, PanBiotech). Viable cells were counted in a Neubauer cytometer using the trypan blue exclusion test. The cells were seeded at a density of 20000 cells/well in 96 well-plates precoated with poly-L-lysine (Batch 3102256, Beckton-Dickinson, France) and laminin (Batch 083M4034V, Sigma-Aldrich France).

The following day of seeding, cells were incubated with or without the compound CR591 of Example 1 (1, 10, 100 pmol/L, 1, 10, 100 nmol/L, 1 and 10 µmol/L diluted in culture medium). The plates were maintained at 37°C in a humidified incubator, in an atmosphere of air (95%)-CO2 (5%). Half of the medium was changed every 2 days with fresh medium in presence or absence of compound.

The following conditions were assessed:

### Plate 1 for Day 12 (A2B5) and plate 2 for Day 18 (MAG evaluation)

- Control (vehicle)
- Compound CR591 of example 1 (1 pmol/L)
- Compound CR591 of example 1 (10 pmol/L)
- Compound CR591 of example 1 (100 pmol/L)
- Compound CR591 of example 1 (1 nmol/L)
- Compound CR591 of example 1 (10 nmol/L)
- Compound CR591 of example 1 (100 nmol/L)
- Compound CR591 of example 1 (1 µmol/L)
- Compound CR591 of example 1 (10 µmol/L)

### b) immunostaining of cells

On days 12 and 18 of culture, cells were fixed by a cold mixture of absolute ethanol 95% (Batch SZBD1470V, Sigma) and acetic acid 5% (Batch SZBD1760V, Sigma) for 5 min. The cells were then permeabilized and non-specific sites were blocked with a solution of phosphate buffered saline (Batch 3010914, PanBiotech) containing 0.1% of saponin (Batch BCBJ8417V, Sigma) and 1% FCS for 15 min at room temperature.

On day 12, cells were incubated with Monoclonal Anti-A2B5 conjugated alexa fluor^{®} 488 produced in mouse (Batch 2281669, Millipore, France) at dilution of 1/200 in PBS containing 1% FCS, 0.1 % saponin, and with anti-MAP-2 antibody produced in chicken (Batch GR180541-3, AbCam; United Kingdom) at dilution of 1/1000 in PBS containing 1% FCS, 0.1 % saponin for 2 h at room temperature. This antibody was revealed with Alexa Fluor 568 goat anti-chicken antibody (Batch 1383072, Molecular probe; France) at the dilution of 1/400 in PBS with 1% FCS, 0.1 % saponin, for 1 h at room temperature.

On day 18, cells were incubated with Monoclonal Anti-MAG antibody produced in mouse (Batch 2301638, Millipore) at dilution of 1/400 in PBS containing 1% FCS, 0.1 % saponin, and with anti-MAP-2 antibody produced in chicken at dilution of 1/1000 in PBS containing 1% FCS, 0.1 % saponin for 2 h at room temperature. These antibodies were revealed with Alexa Fluor 488 goat anti-mouse antibody (Batch 1397999, Molecular probe) at the dilution of 1/400 in PBS with 1% FCS, 0.1 % saponin and Alexa Fluor 568 goat anti-chicken antibody at the dilution of 1/400 in PBS with 1% FCS, 0.1 % saponin, for 1 h at room temperature.

### c) Results

The immunolabeled cultures were automatically examined with ImageXpress (Molecular Devices) equipped with a LED at x20 magnification. For each condition, 30 pictures (representing ~80 % of the total surface of the well) per well were taken. All images were taken with the same conditions. Number of A2B5 positive cells and number of MAG positive cells were automatically analyzed by using Custom module editor (Molecular Devices). Data were expressed in percentage of control conditions (no plant extract = 100 %). All values were expressed as mean +/- SEM (s.e.mean) of the 6 wells. Graphs and statistical analyses are made on the different conditions (ANOVA followed by Dunnett's test when allowed, using GraphPad Prism software).

Treatment with compound CR591 did not show any significant increase of the OPC (precursor cells of oligodendrocytes) number after 12 days treatment (figure 2). At the highest doses, decrease of the total number of OPCs was observed (by an inhibition of the proliferation of the cells or by a toxic effect).

CR591 after 18 days of treatment was able to increase the number of MAG positive cells and significantly improved the myelination of neurons by OL. This effect followed a bell shape curve with a maximal effect for 1 nM. (Figure 3 a and b). Myelin Associated Glycoprotein (MAG) is a specific protein of differentiated oligodendrocytes that is heavily expressed in myelinating oligodendrocytes (Bradl. M., & H. Lassmann., 2010. Acta Neuropathol (2010) 119:37-53).

### Example 4: primary motor neuron culture survival

The aim of this study was to study the effect of CR591 on primary motor neuron culture from rat spinal cord (SC) injured by glutamate exposure (a well validated *in vitro* ALS model and model of motor neuron diseases).

### a) Culture of Spinal cord (SC) motor neurons

Rat SC motor neurons were cultured as described by Martinou et al., Neuron. 1992 Apr;8(4):737-44 and Wang et al., Hum Mol Genet. 2013 Dec 1;22(23):4706-19. Briefly, pregnant female rats (Wistar, Janvier labs) of 14 days gestation were killed by cervical dislocation. Foetuses were collected and immediately placed in ice-cold L15 Leibovitz medium (Batch: 4001014, Pan Biotech) with a 2% penicillin (10,000 U/mL) and streptomycin (10 mg/mL) solution (PS; Pan Biotech, batch: 3090914) and 1% bovine serum albumin (BSA; Pan Biotech, batch: h140603). SC were treated for 20 min at 37°C with a trypsin- EDTA (Pan Biotech, batch: 5890314) solution at a final concentration of 0.05% trypsin and 0.02% EDTA. The dissociation was stopped by addition of Dulbecco's modified Eagle's medium (DMEM) with 4.5 g/liter of glucose (Pan Biotech, batch: 6031214), containing DNAse I grade II (final concentration 0.5 mg/mL; Pan Biotech, batch: H140508) and 10% fetal calf serum (FCS; Invitrogen, batch: 41Q7218K). Cells were mechanically dissociated by three forced passages through the tip of a 10-mL pipette. Cells were then centrifuged at 180g for 10 min at +4°C on a layer of BSA (3.5%) in L15 medium. The supernatant was discarded, and the pellet was resuspended in Neurobasal medium (Invitrogen, batch: 1704746) with a 2% solution of B27 supplement (Invitrogen, batch: 1668967), 2 mmol/L of L-glutamine (Pan Biotech, batch: 6620314), 2% of PS solution, and 10 ng/mL of brain-derived neurotrophic factor (BDNF; Pan Biotech, batch: H140108). Viable cells were counted in a Neubauer cytometer, using the trypan blue exclusion test. The cells were seeded at a density of 20,000 per well in 96-well plates precoated with poly-L-lysine (Biocoat, batch: 21614030) and were cultured at 37°C in an air (95%)-CO2 (5%) incubator. The medium was changed every day.

### b) CR591 and glutamate exposure

On day 13, glutamate was added into cell culture to a final concentration of 5 µM diluted in control medium in presence or absence of the compound CR591 of example 1 for 20 min. After 20 min, the cells were washed- out and new fresh medium containing or not the compound was added for 48h additional time. CR591 (1, 10, 100 pM, 1, 10, 100 nM, 1, 10 µM) was solved and diluted in culture medium and then pre-incubated with primary motor neurons for 1 hour before the glutamate application.

The following conditions were assessed:
- Control
- + glutamate (5 µM 20mn then 48h)
- + glutamate (5 µM 20mn then 48h) + CR591 1 pmol/L
- + glutamate (5 µM 20mn then 48h) + CR591 10 pmol/L
- + glutamate (5 µM 20mn then 48h) + CR591 100 pmol/L
- + glutamate (5 µM 20mn then 48h) + CR591 1 nmol/L
- + glutamate (5 µM 20mn then 48h) + CR591 10 nmol/L
- + glutamate (5 µM 20mn then 48h) + CR591 100 nmol/L
- + glutamate (5 µM 20mn then 48h) + CR591 1 µmol/L
- + glutamate (5 µM 20mn then 48h) + CR591 10 µmol/L

### c) Immunostaining of cells

48 hours after intoxication, the cell culture supernatants were taken off and the SC motor neurons were fixed by a cold solution of ethanol (95%, Sigma, batch: SZBD3080V) and acetic acid (5%, Sigma, Batch: SZBD1760V) for 5 min. After permeabilization with 0.1% of saponin, cells were incubated for 2 hours with an anti microtubule-associated-protein 2 monoclonal antibody (MAP-2; Sigma, batch: (063M4802) at dilution of 1/400 in PBS containing 1% foetal calf serum and 0.1% of saponin. This antibody specifically stains cell bodies of neurons, allowing study of neuron survival in the culture.

This antibody was revealed with Alexa Fluor 488 goat anti-mouse IgG (Molecular probe, batch: 1613346) at the dilution of 1/400 in PBS containing 1% foetal calf serum and 0.1% of saponin for 1 hour at room temperature.

### d) Results

For each condition, 6 wells were assessed, 30 pictures per well were taken using ImageXpress (Molecular Devices) with 20x magnification, to assess motor neuron survival (MAP-2). Analysis of picture was done using Custom Module Editor (Molecular Devices). Results were expressed in terms of mean survival neuron, labeled for MAP-2. Data were expressed in percentage of control conditions (no intoxication, no glutamate = 100 %) in order to express the glutamate injury. All values were expressed as mean +/- SEM (s.e.mean) of the culture (n = 6 wells per condition per culture). Graphs and statistical analyses are made on the different conditions (ANOVA followed by PLSD Fisher's test when allowed, using Statview software version 5.0).

Glutamate (5 µM for 20 min) induced a significant motor neuron death (> 30 %). CR591 added 1h before the glutamate application, displayed a moderate and significant protective effect of motor neuron between 1 nM and 10 nM concentration. (Figure 4).

## Claims

1. Compound of formula (I) 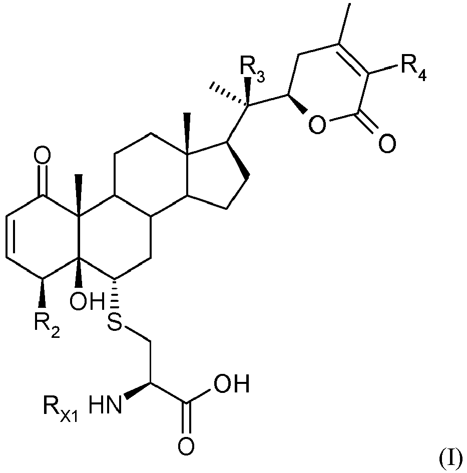 in which
R2 is H, OH, (CH₂)n-CH₃, with n=2, 4 or 6, a glucopyranose or a glucofuranose;
R3 is H, OH, CH₂OH or a glucofuranose;
R4 is H, OH, CH₃, CH₂OH, a glucofuranose, C₆H₅, C₁₀H₇, C₆H₄X or C₁₀H₆X with X= F, Cl, Br or I;
R_{X1} is H or an aminoacid chosen among tryptophane, lysine, methionine, phenylalanine, threonine, valine, leucine, isoleucine, arginine or histidine.

2. Compound according to claim 1 in which
R2 is H,or OH:
R3 is H, OH or CH₂OH;
R4 is H, OH, CH₃, CH₂OH;
R_{X1} is H or an aminoacid chosen among tryptophane, lysine, methionine, phenylalanine, threonine, valine, leucine, isoleucine, arginine or histidine;

3. Compound according to claim 1 or 2 in which
R2 is OH
R3 is H
R4 is CH₂OH
R_{X1} is H;

4. Method of synthesis of the compound of formula (I) according to any of claims 1 to 3, comprising the reaction between compound of formula (II) in which the substituent R1 is H, and R2, R3 and R4 have the same meaning as in formula (I), 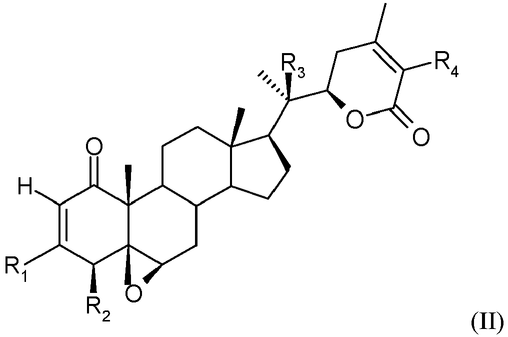 and the compound of formula (III) in which the substituent R_{X1} has the same meaning as in formula (I) 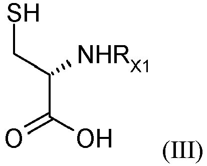 to obtain the compound of formula (I).

5. Method of synthesis according to claim 4, in which the method is made in the presence of a solvent.

6. Method of synthesis according to any of claims 4 or 5, in which the solvent is tetrahydrofurane and/or water.

7. Compound according to any of claims 1 to 3, for its use as medicament.

8. Compound according to claim 7 for its use to treat or limit development of demyelinating diseases in a mammal, preferably a human.

9. Compound for use according to claim 8, in which the demyelinating diseases are chosen among multiple sclerosis, acute disseminated encephalomyelitis, adrenoleukodystrophy, adrenomyeloneuropathy, Leber's Hereditary Optic Atrophy and related mitochondrial disorders, HTLV-associated Myelopathy and diseases linked to demyelination of PNS nerves.

10. Compound according to claim 7 for its use to treat or limit development of neuromuscular diseases in a mammal, preferably a human.

11. Compound for use according to claim 10, in which the neuromuscular diseases are chosen among MN diseases, ALS, PBP, PMA, PLS, SMA, Kennedy's disease, PPS, PPMA, MMN, MMA, paraneoplastic motor neuron disease, LEMS, MG and botulism.

12. Compound for use according to any of claims 7 to 11, wherein said compound is administered orally or parenterally.

13. Compound for use according to any of claims 7 to 11, wherein said compound is administered in a pharmaceutically acceptable vehicle.

## Patentansprüche

1. Verbindung der Formel (I) 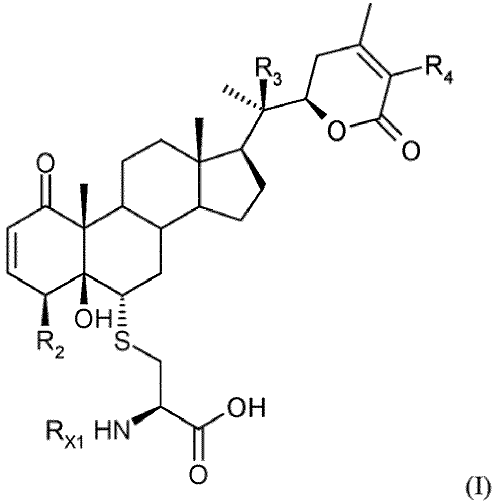 In der
R2 H, OH, (CH₂) n-CH₃, wobei n = 2, 4 oder 6, eine Glucopyranose oder eine Glucofuranose ist;
R3 H, OH, CH₂OH oder eine Glucofuranose ist;
R4 H, OH, CH₃, CH₂OH, eine Glucofuranose, C₆H₅, C₁₀H₇, C₆H₄X oder C₁₀H₆X ist, wobei X = F, Cl, Br oder I;
R_{X1} H oder eine Aminosäure ist, die ausgewählt ist aus Tryptophan, Lysin, Methionin, Phenylalanin, Threonin, Valin, Leucin, Isoleucin, Arginin oder Histidin.

2. Verbindung nach Anspruch 1, in der
R2 H oder OH ist;
R3 H, OH oder CH₂OH ist;
R4 H, OH, CH₃, CH₂OH ist;
R_{X1} H oder eine Aminosäure ist, die ausgewählt ist aus Tryptophan, Lysin, Methionin, Phenylalanin, Threonin, Valin, Leucin, Isoleucin, Arginin oder Histidin.

3. Verbindung nach Anspruch 1 oder 2, in der
R2 OH ist,
R3 H ist,
R4 CH₂OH ist,
R_{X1} H ist.

4. Verfahren zur Synthese der Verbindung der Formel (I) nach einem der Ansprüche 1 bis 3, umfassend die Reaktion zwischen einer Verbindung der Formel (II), in der der Substituent R1 H ist und R2, R3 und R4 die gleiche Bedeutung haben wie in Formel (I), 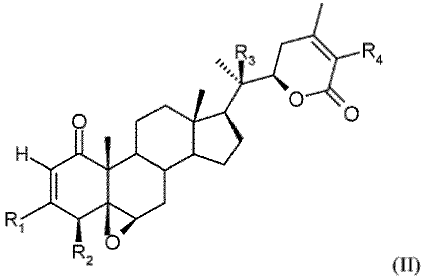 und die Verbindung der Formel (III), in der der Substituent R_{X1} die gleiche Bedeutung hat wie in Formel (I) 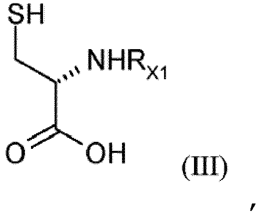 um die Verbindung der Formel (I) zu erhalten.

5. Syntheseverfahren nach Anspruch 4, wobei das Verfahren in Gegenwart eines Lösungsmittels durchgeführt wird.

6. Syntheseverfahren nach einem der Ansprüche 4 oder 5, wobei das Lösungsmittel Tetrahydrofuran und/oder Wasser ist.

7. Verbindung nach einem der Ansprüche 1 bis 3 zur Verwendung als Medikament.

8. Verbindung nach Anspruch 7 zur Verwendung zur Behandlung oder Begrenzung der Entwicklung demyelinisierender Erkrankungen bei einem Säugetier, vorzugsweise einem Menschen.

9. Verbindung zur Verwendung nach Anspruch 8, wobei die demyelinisierenden Erkrankungen ausgewählt sind aus Multipler Sklerose, akuter disseminierter Enzephalomyelitis, Adrenoleukodystrophie, Adrenomyeloneuropathie, Lebersche hereditäre Optikusatrophie und damit verbundene mitochondriale Erkrankungen, HTLV-assoziierte Myelopathie und Erkrankungen im Zusammenhang mit der Demyelinisierung von PNS-Nerven.

10. Verbindung nach Anspruch 7 zur Verwendung zur Behandlung oder Begrenzung der Entwicklung neuromuskulärer Erkrankungen bei einem Säugetier, vorzugsweise einem Menschen.

11. Verbindung zur Verwendung nach Anspruch 10, wobei die neuromuskulären Erkrankungen ausgewählt sind aus MN-Erkrankungen, ALS, PBP, PMA, PLS, SMA, der Kennedy-Krankheit, PPS, PPMA, MMN, MMA, der paraneoplastischen Motoneuron-Erkrankung, LEMS, MG und Botulismus.

12. Verbindung zur Verwendung nach einem der Ansprüche 7 bis 11, wobei die Verbindung oral oder parenteral verabreicht wird.

13. Verbindung zur Verwendung nach einem der Ansprüche 7 bis 11, wobei die Verbindung in einem pharmazeutisch unbedenklichen Träger verabreicht wird.

## Revendications

1. Composé de formule (I) 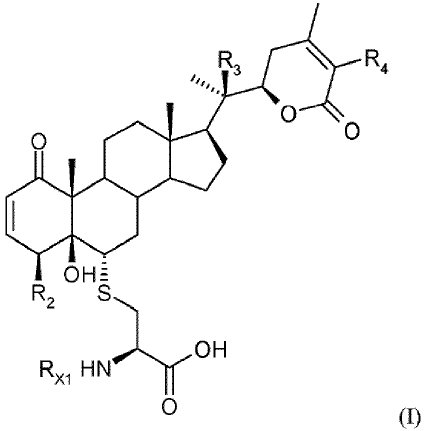 dans lequel
R2 est H, OH, (CH₂)n-CH₃, avec n = 2, 4 ou 6, un glucopyranose ou un glucofuranose ;
R3 est H, OH, CH₂OH ou un glucofuranose ;
R4 est H, OH, CH₃, CH₂OH, un glucofuranose, C₆H₅, C₁₀H₇, C₆H₄X ou C₁₀H₆X avec X = F, Cl, Br ou I ;
R_{X1} est H ou un acide aminé choisi parmi le tryptophane, la lysine, la méthionine, la phénylalanine, la thréonine, la valine, la leucine, l'isoleucine, l'arginine ou l'histidine.

2. Composé selon la revendication 1 dans lequel
R2 est H, ou OH ;
R3 est H, OH ou CH₂OH ;
R4 est H, OH, CH₃, CH₂OH ;
R_{X1} est H ou un acide aminé choisi parmi le tryptophane, la lysine, la méthionine, la phénylalanine, la thréonine, la valine, la leucine, l'isoleucine, l'arginine ou l'histidine.

3. Composé selon la revendication 1 ou 2 dans lequel
R2 est OH
R3 est H
R4 est CH₂OH
R_{X1} est H.

4. Procédé de synthèse du composé de formule (I) selon l'une quelconque des revendications 1 à 3, comprenant la réaction entre le composé de formule (II) dans lequel le substituant R1 est H, et R2, R3 et R4 ont la même signification que dans la formule (I), 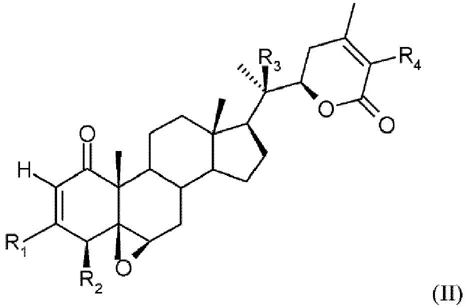 et le composé de formule (III) dans lequel le substituant R_{X1} a la même signification que dans la formule (I) 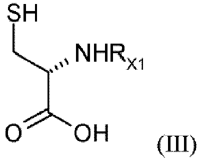 pour obtenir le composé de formule (I).

5. Procédé de synthèse selon la revendication 4, dans lequel le procédé est réalisé en présence d'un solvant.

6. Procédé de synthèse selon l'une quelconque des revendications 4 ou 5, dans lequel le solvant est le tétrahydrofurane et/ou l'eau.

7. Composé selon l'une quelconque des revendications 1 à 3, pour son utilisation en tant que médicament.

8. Composé selon la revendication 7 pour son utilisation pour traiter ou limiter le développement de maladies démyélinisantes chez un mammifère, de préférence un être humain.

9. Composé pour son utilisation selon la revendication 8, dans lequel les maladies démyélinisantes sont choisies parmi une sclérose en plaques, une encéphalomyélite disséminée aiguë, une adrénoleucodystrophie, une adrénomyéloneuropathie, une atrophie optique héréditaire de Leber et les troubles mitochondriaux associés, une myélopathie associée au HTLV et les maladies liées à la démyélinisation des nerfs du SNP.

10. Composé selon la revendication 7 pour son utilisation pour traiter ou limiter le développement de maladies neuromusculaires chez un mammifère, de préférence un humain.

11. Composé pour son utilisation selon la revendication 10, dans lequel les maladies neuromusculaires sont choisies parmi des maladies du MN, une SLA, une PBP, une AMP, un SLP, une SMA, une maladie de Kennedy, un SPP, une AMPP, une NMM, une AM, une maladie du motoneurone paranéoplasique, un SMLE, une MG et un botulisme.

12. Composé pour son utilisation selon l'une quelconque des revendications 7 à 11, dans lequel ledit composé est administré par voie orale ou parentérale.

13. Composé pour son utilisation selon l'une quelconque des revendications 7 à 11, dans lequel ledit composé est administré dans un véhicule pharmaceutiquement acceptable.
